# EUROPEAN PATENT SPECIFICATION

(11) **EP 2 548 029 B1**
(45) Date of publication and mention of the grant of the patent: **04.06.2014**
(21) Application number: 11710381.2
(22) Date of filing: 16.03.2011
(51) Int. Cl.: C12Q 1/37

(54) **METHODS FOR PREDICTING CARDIOVASCULAR EVENTS AND MONITORING TREATMENT USING PCSK9**
VERFAHREN ZUR VORHERSAGE KARDIOVASKULÄREN EREIGNISSE UND ZUR ÜBERWACHUNG DER BEHANDLUNG MITTELS PCSK9
PROCÉDÉS DE PRÉDICTION D'ÉVÉNEMENTS CARDIOVASCULAIRES ET DE SURVEILLANCE DU TRAITEMENT EN UTILISANT PCSK9

(30) Priority: 16.03.2010 US 314316 P
(43) Date of publication of application: 23.01.2013
(73) Proprietor: BG Medicine, Inc., Waltham, MA 02451 (US)
(72) Inventor: MUNTENDAM, Pieter, Boxford, MA 01921 (US)
(74) Representative: D'Arcy, Julia
(86) International application number: PCT/US2011/028721
(87) International publication number: WO 2011/116128

(56) References cited:
- WO-A1-2007/128121
- WO-A2-2008/105797
- LAMBERT GILLES ET AL: "Plasma PCSK9 concentrations correlate with LDL and total cholesterol in diabetic patients and are decreased by fenofibrate treatment", CLINICAL CHEMISTRY, AMERICAN ASSOCIATION FOR CLINICAL CHEMISTRY, WASHINGTON, DC, vol. 54, no. 6, 1 June 2008 (2008-06-01), pages 1038-1045, XP009104835, ISSN: 0009-9147, DOI: DOI:10.1373/CLINCHEM.2007.099747
- DUBUC GENEVIEVE ET AL: "A new method for measurement of total plasma PCSK9: clinical applications", JOURNAL OF LIPID RESEARCH, vol. 51, no. 1, January 2010 (2010-01), pages 140-149, XP002633970, ISSN: 0022-2275
- CARESKEY H E ET AL: "Atorvastatin increases human serum levels of proprotein convertase subtilisin/kexin type 9", JOURNAL OF LIPID RESEARCH 200802 US LNKD- DOI:10.1194/JLR.M700437-JLR200, vol. 49, no. 2, February 2008 (2008-02), pages 394-398, XP002633971, ISSN: 0022-2275
- LAKOSKI SUSAN G ET AL: "Indices of cholesterol metabolism and relative responsiveness to ezetimibe and simvastatin.", THE JOURNAL OF CLINICAL ENDOCRINOLOGY AND METABOLISM FEB 2010 LNKD- PUBMED:19965915, vol. 95, no. 2, February 2010 (2010-02), pages 800-809, XP002633972, ISSN: 1945-7197

## Description

### BACKGROUND

Atherosclerotic cardiovascular disease (CVD) and cardiovascular events (CVE) including, for example, myocardial infarction (MI), are predominantly caused by modifiable risk factors but nonetheless remains the leading cause of death and severe disability worldwide. (Yusuf et al., Effect of potentially modifiable risk factors associated with myocardial infarction in 52 countries (the INTERHEART study): case-control study, Lancet, 364:937-52 (2004)) To prevent the disease, contemporary American and European guidelines recommend an integrated two-step approach in which risk assessment (prediction) is followed by individualized risk reduction (therapy), if needed; the higher the risk, the more aggressive the prescribed preventive care. (Third Report of the National Cholesterol Education Program (NCEP) Expert Panel on Detection, Evaluation, and Treatment of High Blood Cholesterol in Adults (Adult Treatment Panel III) final report, Circulation, 106:3143-421 (2002); European guidelines on cardiovascular disease prevention in clinical practice: executive summary, Eur. Heart J., 28:2375-414 (2007)).

Risk assessment in primary prevention of atherosclerotic cardiovascular disease has not changed dramatically in the last 40 years. It remains based upon the risk factor concept introduced by the Framingham Heart Study in the 1960's. (Kannel et al., Factors of risk in the development of coronary heart disease--six year follow-up experience: The Framingham Study, Ann. Intern. Med., 55:33-50 (1961)). Because individual risk factors such as plasma cholesterol and blood pressure have low independent predictive ability (Ware. The limitations of risk factors as prognostic tools, N. Engl. J. Med., 355:2615-7 (2006)), they have been combined to generate global risk assessment measures such as the Framingham Risk Score (FRS) and the European SCORE (Systematic Coronary Risk Evaluation). (Third Report of the National Cholesterol Education Program (NCEP) Expert Panel on Detection, Evaluation, and Treatment of High Blood Cholesterol in Adults (Adult Treatment Panel III) final report, Circulation, 106:3143-421 (2002); European guidelines on cardiovascular disease prevention in clinical practice: executive summary, Eur. Heart J., 28:2375-414 (2007)).

In addition, the medical practice of administration of pharmacological agents to individuals with cardiovascular disease, such as hyperlipidemia, has revealed treatment responses that differ among different individuals, or at different times, or with different dosages. For example, HMG-CoA reductase inhibitors, or statins, are a class of hypolipidemic drugs that are prescribed to treat hyperlipidemia and reduce cardiovascular disease risk by lowering blood total cholesterol levels in humans by inhibiting the enzyme HMG-CoA reductase. Inhibition of this enzyme decreases cholesterol synthesis in the liver and increases low-density lipoprotein (LDL) receptors, resulting in an increased clearance of LDL from circulation. Individual response to statins may be assessed by measurement of blood concentrations of lipids and/or lipoproteins. It has been found that individual response to statins is highly variable. It has been postulated that genetic variation may modify both statin efficacy and susceptibility to statin-induced adverse drug reactions; however, definitive genetic variations associated with statin response have been elusive (Mangravite et al., Pharmacogenomics of statin response, Curr. Opin. Mol. Ther., 10(6):555-61 (2008)) Similar varying treatment responses characterize other pharmacological therapies for cardiovascular disease, such as peroxisome proliferator activated receptor alpha agonists, or fibrates.

The scientific article entitled, "Plasma PCSK9 concentrations correlate with LDL and total cholesterol in diabetic patients and are decreased by fenofibrate treatment," (Lambert Gilles et al., Clinical Chemistry, American Association for Clinical Chemistry, Washington DC, vol. 54, no. 6, 1 June 2008, pages 1038 to 1045 XP009104835) discloses an ELISA for measuring PCSK9 levels in blood samples of cardiovascular patients. This article associates a decrease in PCSK9 with at least some of the LDL cholesterol-lowering effects of fenofibrate.

The scientific article entitled, "A new method for measurement of total plasma PCSK9: clinical applications," (Dubuc et al., Journal of Lipid Research, vol. 51, no. 1, January 2010 (2010-01), pages 140 to 149) describes the development of a polyclonal antibody against human PCSK9 and its use to develop an ELISA.

The scientific article entitled, "Atorvastatin increases human serum levels of proprotein convertase subtilisin/kexin type 9," (Careskey et al., Journal of Lipid Research, vol. 49, no. 2, February 2008 (2008-02), pages 394-398) provides a study of the effect of atorvastatin on circulating PCSK9 protein levels in humans using a sandwich ELISA. The increase of circulating PCSK9 levels with atorvastatin is highlighted.

The scientific article entitled, "Indices of cholesterol metabolism and relative responsiveness to ezetimibe and simvastatin," (Lakosi et al., The Journal of Clinical Endocrinology and Metabolism, vol. 95, no. 2, February 2010 (2010-02), pages 800 to 809 tests the hypothesis that individuals with high fractional absorption of cholesterol respond better to the cholesterol absorption inhibitor ezetimibe than to simvastatin, and that low absorbers who have elevated rates of cholesterol synthesis respond better to simvastatin. This article discloses that responsiveness to simvastatin and ezetimibe is highly correlated.

Polynucleotides encoding PCSK9b and PCSK9c polypeptides, fragments and homologues thereof are disclosed in International Publication No. WO 2008/105797. Diagnostic and therapeutic methods are also provided for applying these polypeptides to the diagnosis, treatment, and/or prevention of various diseases and/or disorders related to these polypeptides. This document also discloses screening methods for identifying agonists and antagonists of the polynucleotides and polypeptides disclosed therein.

International Publication No. WO 2007/128121 discloses proteins comprising a PCSK9 sequence, and cells expressing such a protein. A method of selecting a treatment for a subject comprising measuring the concentration of a specific polypeptide in a blood sample of the subject, wherein a concentration of the polypeptide higher in the blood sample of the subject than that in a control blood sample is an indication that a PCSK9 inhibitor may be a useful treatment for the subject.

A medically unsatisfactory pharmacological treatment effect for an individual with any pharmacological treatment for cardiovascluar disease and/or hyperlipidemia may result in a physician changing prescribed dosage and/or frequency, or attempting an alternate therapeutic strategy. Physicians routinely prescribe treatment regimens without knowledge of how an individual will respond to the treatment. As such, a trial and error treatment strategy is initiated, often at great cost and at the expense of severe side effects and loss of valuable treatment time.

Thus, there is a need to improve the prediction of individual response to pharmacological therapies for hyperlipidemia and cardiovascular disease.

### SUMMARY

It has been discovered that proprotein convertase subtilisin kexin type 9 (PCSK9) levels can be used to evaluate a patient's expected response to drug treatment for cardiovascular disease (e.g., hyperlipidemia or high cholesterol). For example, a patient's PCSK9 levels can be measured prior to drug administration (or after a sufficient washout period) to determine whether the patient is likely to respond to a statin.

A method is provided for predicting response to treatment with a statin. The method includes measuring a proprotein convertase subtilisin kexin type 9 (PCSK9) level in a sample obtained from a patient prior to the patient taking a statin or after a sufficient washout period (e.g., about 6 weeks) has elapsed for the patient. A measured PCSK9 level falling within a predetermined target range or under a maximum threshold can be indicative that the patient is likely to respond favorably to treatment with a statin. On the other hand, a measured PCSK9 level above the predetermined target range (e.g., about 0 nanomoles per liter to about 7 nanomoles per liter) or a maximum threshold can be indicative that the patient is likely to have an attenuated response to treatment with a statin. In some embodiments, the measured PCSK9 level can be an absolute concentration or it can be a normalized concentration.

In methods of the present teachings, non-limiting examples of statins include: atorvastatin, cerivastatin, fluvastatin, lovastatin, mevastatin, pitavastatin, pravastatin, rosuvastatin, and simvastatin.

A computer system also is provided for predicting patient response to treatment with a statin. The computer system includes an electronic memory device and an electronic processor in communication with the memory device. The memory device includes instructions that when executed by the processor cause the processor to: query a proprotein convertase subtilisin kexin type 9 (PCSK9) level measured in a sample obtained from a patient prior to the patient taking a statin or after a sufficient washout period has elapsed for the patient; compare the measured PCSK9 level to a threshold range; and generate a notification indicative of whether the measured PCSK9 level is within the target range. A measured PCSK9 level falling within a predetermined target range can be indicative that the patient is likely to respond favorably to treatment with a statin. A measured PCSK9 level above the predetermined range can be indicative that the patient is likely to have an attenuated response to treatment with a statin.

In methods of the present teachings, the sample can include blood, serum or plasma.

In methods of the present teachings, the patient can have a PCSK9 blood concentration determined to be within a target range (e.g., about 0 nanomoles per liter to about 7 nanomoles per liter). In some embodiments, the patient can have a PCSK9 blood concentration determined to be above a minimum threshold. The minimum threshold can be, for example, more than 1 nanomole per liter, between 1 and 1.5 nanomoles per liter, between 1.5 and 2 nanomoles per liter, between 2 and 2.5 nanomoles per liter, between 2.5 and 3 nanomoles per liter, and/or more than 3 nanomoles per liter. In some embodiments, the patient can have a PCSK9 blood concentration determined to be below a maximum threshold. The maximum threshold can be, for example, below 7 nanomoles per liter, below 6 nanomole per liter, below 4 nanomoles per liter, between 3 and 4 nanomoles per liter, between 2.5 and 3 nanomoles per liter, between 2 and 2.5 nanomoles per liter, and/or between 1.5 and 2 nanomoles per liter.

### BRIEF DESCRIPTION OF DRAWING

The present teachings described herein will be more fully understood from the following description of various illustrative embodiments, when read together with the accompanying drawing. It should be understood that the drawing described below is for illustration purposes only and is not intended to limit the scope of the present teachings in any way.

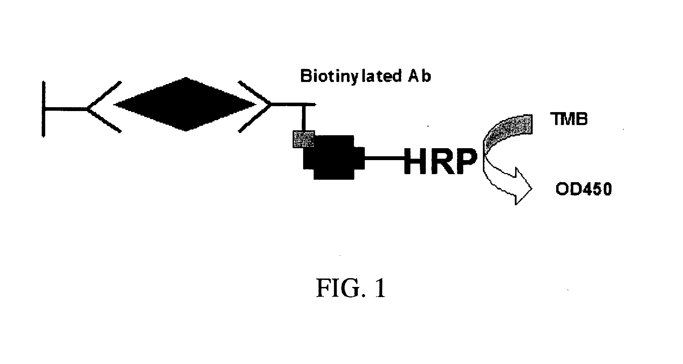

FIG. 1 is a schematic of a colorimetric assay for determining PCSK9 concentration, in accordance with an illustrative embodiment.

### DETAILED DESCRIPTION

It has been discovered that PCSK9 levels can be used to evaluate a patient's expected response to drug treatment for cardiovascular disease (e.g., hyperlipidemia or high cholesterol). For example, a patient's PCSK9 levels can be measured prior to drug administration (or after a sufficient washout period) to determine whether the patient is likely to respond to a statin. A patient whose PCSK9 levels fall within a target range has an increased likelihood of responding favorably to the selected treatment. Thus, physicians and patients can use this information to make informed decisions before selecting a treatment regimen.

In various embodiments, PCSK9 levels in a patient are used as a biomarker. In general, a "biomarker" can be any biological feature or variable whose qualitative or quantitative presence, absence, or level in a biological system such as a human is an indicator of a biological state of the system. Accordingly, biomarkers can be useful to assess the health state or status of an individual by comparing the measured level of one or more biomarkers in a patient or a patient sample to a control.

As described in detail in the Examples, the present teachings provide methods for predicting whether a patient will respond favorably to a statin prior to administering the statin. This information can allow physicians to more quickly select appropriate treatments, which potentially can save lives and also can reduce resources spent on ineffective or not optimally effective treatments.

As described above, the term "biomarker" refers to any biological feature or variable whose qualitative or quantitative presence, absence, or level in a biological system such as a human is an indicator of a biological state of the system. For example, a biomarker of an organism can be useful, alone or in combination with other biomarkers and/or clinical risk factors, to measure the initiation, progression, severity, pathology, aggressiveness, grade, activity, disability, mortality, morbidity, disease sub-classification or other underlying feature of one or more biological processes, pathogenic processes, diseases, or responses to therapeutic intervention. Virtually any biological compound that is present in a sample and that can be isolated from, or measured in, the sample can be used as a biomarker. Non-limiting examples of classes of biomarkers include a polypeptide, a proteoglycan, a glycoprotein, a lipoprotein, a carbohydrate, a lipid, a nucleic acid, an organic on inorganic chemical, a natural polymer, a metabolite, and a small molecule. A biomarker also can include a physical measurement of the human body, such as blood pressure and cell counts, as well as the ratio or proportion of two or more biological features or variables.

The "level" or "amount" of a biomarker can be determined by any method known in the art and will depend in part on the nature of the biomarker. It should be understood that the amount of the biomarker need not be determined in absolute terms, but can be determined in relative terms. Thus, in some embodiments, (measured) levels can be absolute concentrations measured in a sample obtained from a patient, and in some embodiments, (measured) levels can be normalized concentrations measured in a sample obtained from a patient. In addition, the amount of the biomarker can be expressed by its concentration in a biological sample, for example, a sample obtained from a mammal such as a human, by the concentration of an antibody that binds to the biomarker, or by the functional activity (i.e., binding or enzymatic activity) of the biomarker.

The terms "reference," "control" and "standard" can refer to an amount of a biomarker in a healthy individual or a control population or to a risk score derived from one or more biomarkers in a healthy individual or a control population. The amount of a biomarker can be determined in a sample of a healthy individual, or can be determined in samples of a control population. A control population can be a group of healthy individuals, individuals lacking diagnosis of the particular disease for which the biomarker is indicative, and/or a sub-population of such individuals where the sub-population is selected based on the background of the patient, for example, based on gender, age, ethnicity, or other distinguishing or clinically relevant features.

The term "sample" refers to any biological sample from an individual (e.g., a patient), including body fluids, blood, blood plasma, blood serum, sebum, cerebrospinal fluid, bile acid, saliva, synovial fluid, pleural fluid, pericardial fluid, peritoneal fluid, sweat, feces, nasal fluid, ocular fluid, intracellular fluid, intercellular fluid, lymph urine, tissue, liver cells, epithelial cells, endothelial cells, kidney cells, prostate cells, blood cells, lung cells, brain cells, adipose cells, tumor cells, and mammary cells. The sources of biological sample types can be different subjects; the same subject at different times; the same subject in different states, e.g., prior to drug treatment and after drug treatment; different sexes; different species, for example, a human and a non-human mammal; and various other permutations. Further, a biological sample type can be treated differently prior to evaluation such as using different work-up protocols.

The present teachings also provide methods of predicting response to treatment with a statin (e.g., statin treatment for high cholesterol), including predicting whether the treatment is suitable for a patient prior to administration thereof or after a sufficient washout period (e.g., 6-8 weeks without the therapy). In some embodiments, the method includes measuring a PCSK9 blood concentration in a sample from the patient to determine the presence or absence of a PCSK9 blood concentration indicative of responsiveness to a particular therapy. If the patient's PCSK9 level indicates that the patient may respond favorably to a particular statin therapy, then that therapy can be prescribed or administered with higher confidence. If the patient's PCSK9 level indicates that the patient may not respond or may respond unfavorably to the therapy, then an alternative therapy can be prescribed or administered.

Patients who have a PCSK9 blood concentration within a target range are considered to have a higher likelihood of responding favorably to a therapy. The target range can be, for example, between about 0 nanomole per liter (nM) to about 7 nM. In various embodiments, the target range has a minimum threshold of more than about 0 nM, more than about 0.5 nM, more than about 1 nM, between about 1-1.5 nM, between about 1-2 nM, between about 2 nM to about 2.5 nM, between about 2.5 nM to about 3 nM, or about 3 nM. In various embodiments, the target range has a maximum threshold of less than about 7 nM, about 6 nM, about 5 nM, about 4 nM, between about 3 nM to about 4 nM, between about 2.5 nM to about 3 nM, between about 2 nM to about 2.5 nM, between about 1.5 nM to about 2 nM or about 1.5 nM.

Measured PCSK9 levels can be absolute PCSK9 concentrations measured in a sample obtained from a patient, or measured PCSK9 levels can be normalized PCSK9 concentrations measured in a sample obtained from a patient.

The measured levels of PCSK9 can be indicative of whether a patient will respond to a therapy including a statin. Suitable statins include, by non-limiting example, atorvastatin, cerivastatin, fluvastatin, lovastatin, mevastatin, pitavastatin, pravastatin, rosuvastatin, and simvastatin. In certain embodiments, the statin is rosuvastatin. In some embodiments, the statin is atorvastatin.

After it is determined that a patient is likely to respond favorably to therapy including administration of a statin, the therapy can be administered in an amount sufficient to inhibit progression or development of cardiovascular disease and/or a cardiovascular event and, preferably, in a survival-enhancing amount. In addition, the dosage and/or frequency of administration can be increased or decreased based on the patient's PCSK9 levels.

Any of the methods disclosed herein can be embodied as or into systems, for example, computer systems. A computer system can include an electronic memory device (e.g., a computer readable medium) and an electronic processor in communication with the memory device. The electronic memory device includes instructions for carrying out the disclosed methods. Such systems would be beneficial to healthcare providers and insurers, both for efficiently managing and tracking patient data and for managing patients.

The present teachings provide methods where PCSK9 levels are measured. Many methods for detecting expression levels of a protein of interest, with or without quantitation, are well known and can be used with the present teachings. Examples of such assays are described below and can include, for example, immunoassays, chromatographic methods, and mass spectroscopy. Such assays can be performed on any biological sample including, among others, blood, plasma, and serum. In addition, many methods for detecting expression levels of a gene transcript (e.g., mRNA) of interest, with or without quantitation, are will known and can be used with the present teachings.

Biomarkers can be detected or quantified in a sample with the help of one or more "separation" analytical methods. For example, suitable separation methods can include a mass spectrometry method, such as electrospray ionization mass spectrometry (ESI-MS), ESI-MS/MS, ESI-MS/(MS)n (n is an integer greater than zero), matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-(MS)n, or atmospheric pressure photoionization mass spectrometry (APPI-MS), APPI-MS/MS, and APPI-(MS)n. Other mass spectrometry methods can include, for example, quadrupole, fourier transform mass spectrometry (FTMS) and ion trap. Spectrometric techniques that can also be used include resonance spectroscopy and optical spectroscopy.

Other suitable separation methods include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE), or other chromatographic techniques, such as thin-layer, gas or liquid chromatography, or any combination thereof. In some embodiments, the biological sample to be assayed can be fractionated prior to application of the separation analytical technique.

Biomarkers can be detected or quantified by methods that do not require physical separation of the biomarkers themselves. For example, nuclear magnetic resonance (NMR) spectroscopy can be used to resolve a profile of a biomarker from a complex mixture of molecules. An analogous use of NMR to classify tumors is disclosed in Hagberg, NMR Biomed., 11:148-56 (1998), for example.

A biomarker in a sample also can be detected or quantified by combining the biomarker with a binding moiety capable of specifically binding the biomarker. The binding moiety can include a member of a ligand-receptor pair, i.e., a pair of molecules capable of having a specific binding interaction. The binding moiety also can include a member of a specific binding pair, such as antibody-antigen, enzyme-substrate, nucleic acid-nucleic acid, protein-nucleic acid, protein-protein, or other specific binding pairs known in the art. Binding proteins can be designed that have enhanced affinity for a target. Optionally, the binding moiety can be linked with a detectable label, such as an enzymatic, fluorescent, radioactive, phosphorescent or colored particle label. The labeled complex can be detected, e.g., visually or with the aid of a spectrophotometer or other detector, and/or can be quantified.

For example, PCSK9 can be detected using an immunoassay, such as an enzyme-linked immunosorbant assay (ELISA). An immunoassay can be performed by contacting a sample from a subject to be tested with an appropriate antibody under conditions such that immunospecific binding can occur if the biomarker is present. Subsequently, detecting and/or measuring the amount of any immunospecific binding by the antibody to the biomarker can be done. Other suitable immunoassays can be used, including, without limitation, competitive and non-competitive assay systems using techniques such as Western blots, radioimmunoassays, "sandwich" immunoassays, immunoprecipitation assays, immunodiffusion assays, agglutination assays, complement-fixation assays, immunoradiometric assays, and fluorescent immunoassays.

In a sandwich immunoassay, two antibodies capable of binding a biomarker generally are used, e.g., one immobilized onto a solid support, and one free in solution and labeled with a detectable chemical compound. Examples of chemical labels that can be used for the second antibody include radioisotopes, fluorescent compounds, and enzymes or other molecules that generate colored or electrochemically active products when exposed to a reactant or enzyme substrate. When a sample containing the biomarker is placed in this assaying system, the biomarker can bind to both the immobilized antibody and the labeled antibody, to form a "sandwich" complex on the support's surface. The complexed biomarker can be detected by washing away non-bound sample components and excess labeled antibody, and measuring the amount of labeled antibody complexed to the biomarker on the support's surface. Alternatively, the antibody which is free in solution can be labeled with a chemical moiety, for example, a hapten, which can be detected by a third antibody labeled with a detectable moiety that binds the free antibody or, for example, the hapten coupled thereto.

Both the sandwich immunoassay and tissue immunohistochemical procedures can be highly specific and very sensitive, provided that labels with good limits of detection are used. A detailed review of immunological assay design, theory and protocols can be found in numerous texts in the art, including Butt, Practical Immunology (ed. Marcel Dekker, New York (1984)) and Harlow et al. Antibodies. A Laboratory Approach (ed. Cold Spring Harbor Laboratory (1988)).

In general, immunoassay design considerations include preparation of antibodies (e.g., monoclonal or polyclonal antibodies) having sufficiently high binding specificity for the target to form a complex that can be distinguished reliably from products of nonspecific interactions. As used herein, the term "antibody" is understood to mean binding proteins, for example, antibodies or other proteins comprising an immunoglobulin variable region-like binding domain, having the appropriate binding affinities and specificities for the target. The higher the antibody binding specificity, the lower the target concentration that can be detected. As used herein, the terms "specific binding" or "binding specifically" are understood to mean that the binding moiety, for example, a binding protein, has a binding affinity for the target of greater than about 10⁵ M⁻¹, and preferably greater than about 10⁷ M⁻¹.

In the present teachings, when an immunoassay is used to determine the level of PCSK9 present in a sample from a patient, the antibodies used in the assay can include, for example, H1 H316P, H1 M300N, H1 H313, H1 H314, H1 H315, H1 H317, H1 H318, H1 H320, H1 H321, H1 H334 (as described in International Application No. PCT/US2009/068013); KS-2C10 (Circulex Human PCSK9 ELISA Kit, Cat# CY-8079; Nagano, Japan); AX1, AX213, AX214; 3BX5C01, 3CX2A06, 3CX3D02, 3CX4B08 (as described in International Application No. PCT/US2007/023213); EB06682 (Everest Biotech, Oxfordshire, United Kingdon); ab28770, ab31762, ab52754, ab52755, ab95478, ab92753, ab42086, ab42085 (Abcam, Cambridge, MA); antibodies as described in Alborn et al., "Serum proprotein convertase subtilisin kexin type 9 is correlated directly with serum LDL cholesterol," 53(10):1814-9 (2007). Moreover, one skilled in the art knows how to make or raise antibodies to a known target.

Target gene transcripts can be detected using numerous techniques that are well known in the art. Some useful nucleic acid detection systems involve preparing a purified nucleic acid fraction of a sample (e.g., a tumor biopsy, a cancer cell culture, a cell engrafted biocompatible matrix) and subjecting the sample to a direct detection assay or an amplification process followed by a detection assay. Amplification can be achieved, for example, by polymerase chain reaction (PCR), reverse transcriptase (RT), and coupled RT-PCR. Detection of a nucleic acid can be accomplished, for example, by probing the purified nucleic acid fraction with a probe that hybridizes to the nucleic acid of interest and in many instances, detection involves an amplification as well. Northern blots, dot blots, microarrays, quantitative PCR, quantitative RT-PCR, and real-time PCR are all well known methods for detecting a nucleic acid in a sample. Nucleic acids also can be amplified by ligase chain reaction, strand displacement amplification, self-sustained sequence replication or nucleic acid sequence-based amplification. Nucleic acids also can be detected by sequencing. The sequencing can use a primer specific to the target nucleic acid or a primer to an adaptor sequence attached to the target nucleic acid. Sequencing of randomly selected mRNA or cDNA sequences can provide an indication of the relative expression of a biomarker as indicated by the percentage of all sequenced transcripts containing the nucleic acid sequence corresponding to the biomarker.

Alternatively, a nucleic acid can be detected *in situ,* such as by hybridization, without extraction or purification. Gene transcripts can be detected on a medium-throughput basis, such as by using a qRT-PCR array (e.g., RT2 Endothelial Cell Biology PCR Array; SABiosciences, Baltimore, MD). In addition, target gene transcripts can be detected on a high-thoughput basis using a number of well known methods, such as cDNA microarrays (Affymetrix, Santa Clara, CA), SAGE (Invitrogen, Carlsbad, CA), and high-throughput mRNA sequencing (Illumina Inc., San Diego, CA).

The present teachings are further illustrated by the following examples, which are provided for illustration and not limitation.

### EXAMPLE 1: Experimental Design.

Concentrations of PCSK9 protein were measured in blood serum specimens of human subjects diagnosed with primary hypercholesterolemia or mixed hyperlipidemia who had participated in a multicenter, randomized, double-blind study to evaluate the lipid-altering efficacy and safety of pharmacological agents. Briefly, following a 6 to 8 week washout period, subjects were randomized to seven treatment arms: (i) 10 milligrams (mg) simvastatin administered daily for 4 weeks, followed by 20 mg simvastatin administered daily for 8 weeks; (ii) 20 mg simvastatin administered daily for 4 weeks, followed by 40 mg simvastatin administered daily for 8 weeks; (iii) 40 mg simvastatin administered daily for 12 weeks; (iv) 10 mg simvastatin co-administered with 1 g niacin and 20 mg laropiprant daily for 4 weeks, followed by 20 mg simvastatin co-administered with 2 g niacin and 40 mg laropiprant daily for 8 weeks; (v) 20 mg simvastatin co-administered with 1 g niacin and 20 mg laropiprant daily for 4 weeks, followed by 40 mg simvastatin co-administered with 2 g niacin and 40 mg laropiprant daily for 8 weeks; (vi) 40 mg simvastatin co-administered with 1 g niacin and 20 mg laropiprant daily for 4 weeks, followed by 40 mg simvastatin co-administered with 2 g niacin and 40 mg laropiprant daily for 8 weeks, and (vii) 1 g niacin and 20 mg laropiprant daily for 4 weeks, followed by 2 g niacin and 40 mg laropiprant daily for 8 weeks (with no simvastatin administration). Simvastatin, as all statins as a drug class, acts by inhibiting 3-hydroxy-3-methyl-glutaryl-CoA reductase, thereby modulating a biochemical pathway involved in the hepatic synthesis of cholesterol.

Levels of PCSK9 were assessed in blood serum specimens acquired just prior the start of the therapeutic administration (referred to as `Week 0') and at 12 weeks after the end of the therapeutic administration period (referred to as 'Week 12'). Approximately 50 to 75 subjects assigned to each of the seven treatment arms in the study provided blood samples.

Blood serum PCSK9 concentration was determined using a colorimetric assay. The PCSK9 concentrated calibrator (7.5 mg/mL), and the assay antibodies AX213 and AX1-Biotin were the reagents for the assay.

A concentration of 7.5 mg/mL assigned to the PCSK9 concentrated calibrator was used for calculating the needed dilution factors for preparing the calibrators used in the PCSK9 assay. A conversion factor of 1/78 was used to convert ng/mL to nM assuming a molecular weight for PCSK9 of 78,000 Daltons. Figure 1 shows the colorimetric based detection system using horseradish peroxidase (HRP) enzyme chemistry for measuring PCSK9 in clinical samples. It takes advantage of the antibody pair as well as the binding reaction conditions. After adding the tetramethylbenzidine (TMB) substrate, the color development is measured by optical absorbance at a wavelength of 450 nm. Color development can be stopped by adding 100 microliters of 0.5M sulfuric acid.

Assay plates were prepared each day by adding 60 microliters of coating solution per well and incubated overnight at 4 °C. Coating solution included 8.4 micrograms/mL of the capture antibody, AX213, in PBS buffer. The next morning, prior to use, they were blocked with 150 microliters of blocking solution (TBS buffer with 3% BSA and 0.05% Tweeen-20) per well for 1 hour at room temperature. Samples were diluted 100-fold in the assay diluent and measured in duplicate. Diluted samples, diluted controls, and calibrators were added into corresponding wells in the microtiter plate (50 µL per well) and incubated in the Boekel Jitterbug shaker (Boekel Scientific, Feasterville, PA) with shaking for 1 hour at 37 °C. After washing with the wash buffer (400 µL per cycle for 4 cycles), 50 microliters of the biotinlyated detection antibody, AX1-Biotin, was added to each well at a final concentration of 1.0 µg/mL. The plate was incubated for 1 hour with shaking at room temperature followed by washing. 100 µL of Streptavidin-HRP conjugate was added into each well (10 ng/mL). The plate was incubated for 30 minutes without shaking at room temperature and was subsequently washed for 4 cycles with 400 µL of the wash buffer. 100 µL of TMB substrate was added into each well. The plate was incubated without shaking at room temperature for 20 minutes. After adding 100 µL of the stop solution, the plate was read at 450 nm.

### EXAMPLE 2: Baseline PCSK9 levels are indicative of therapeutic response to treatment with a statin.

Samples and data were collected as described in Example 1.

It was found that subjects with a higher level of PCSK9 at Week 0 were associated with clinically poorer response to therapy with a statin for primary hypercholesterolemia or mixed hyperlipidemia. The majority of subjects exhibited clinically beneficial changes in blood parameters between Week 0 and Week 12, increases in levels of high density lipoprotein cholesterol (HDL-C) and apolipoprotein A1, and such as decreases in levels of low density lipoprotein cholesterol (LDL-C) and apolipoprotein B. However, it was found that subjects with high levels of PCSK9 at Week 0 exhibited relatively more modest increases in HDL-C and apolipoprotein A1 and relatively more modest decreases in LDL-C and apolipoprotein B.

A separate regression model for each of the parameters of HDL-C, LDL-C, apolipoprotein A1 and apolipoprotein B was evaluated. In each regression model, percent change in PCSK9 from Week 0 to Week 12 was on the left side of the equation, and on the right side of the equation each such regression model included the factors of PCSK9 at Week 0, treatment group, gender, age (as a continuous variable), geographical region (United States or non-United States), and Week 0 value of the parameter (i.e. of HDL-C, LDL-C, apolipoprotein A1 and apolipoprotein B; whichever was being evaluated). For this exercise, the following two treatment groups were combined: (i) 20 mg simvastatin administered daily for 4 weeks, followed by 40 mg simvastatin administered daily for 8 weeks (N=67), and (ii) 40 mg simvastatin administered daily for 12 weeks (N=71). Table 1 summarizes the results of these regression analyses.

**Table 1. Regression coefficient (slope) of Week 0 PCSK9 level for prediction of percent change from Week 0 to Week 12 in the indicated parameter.**

| Parameter | Slope (Standard Error) | 95% Confidence Interval |
|---|---|---|
| HDL-C | -2.59 (1.00) | -4.56 to -0.63 |
| apolipoprotein B | +1.93 (0.95) | +0.06 to +3.80 |
| LDL-C | +1.38 (1.05) | -0.69 to +3.45 |
| apolipoprotein A1 | -1.49 (1.10) | -3.64 to +0.67 |

From the table, it is observed that for every 1 nanomolar (nM) increase in PCSK9 level at Week 0, the increase from Week 0 to Week 12 in HDL-C is attenuated by 2.59%, after controlling for the other factors included in the regression model. Because a larger increase in HDL-C is generally clinically desirable for treatment of hypercholesterolemia and hyperlipidemia, these results indicate that higher baseline values or levels of PCSK9 are associated with poorer response to the pharmacological statin therapy. Such information can inform a physician or health care professional in therapeutic decisions of drug dosage and/or frequency, drug selection, and the like. Similarly, from the table, it is observed that for every 1 nM increase in PCSK9 level at Week 0, the decrease from Week 0 to Week 12 in LDL-C is attenuated by 1.38%, after controlling for the other factors included in the regression model. Because a larger decrease in LDL-C is generally desirable clinically for hypercholesterolemia and hyperlipidemia, these results also indicate that higher baseline values or levels of PCSK9 are associated with poorer response to the pharmacological statin therapy.

The use of headings and sections in the application is not meant to limit the present teachings; each section can apply to any aspect, embodiment, or feature of the present teachings.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components, or can be selected from a group consisting of two or more of the recited elements or components. Further, it should be understood that elements and/or features of a composition, an apparatus, or a method described herein can be combined in a variety of ways, whether explicit or implicit herein.

The use of the terms "include," "includes," "including," "have," "has," "having," "contain," "contains," or "containing" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise. In addition, where the use of the term "about" is before a quantitative value, the present teachings also include the specific quantitative value itself, unless specifically stated otherwise. As used herein, the term "about" refers to a ±10% variation from the nominal value, unless otherwise indicated or inferred.

It should be understood that the order of steps or order for performing certain actions is immaterial so long as the present teachings remain operable. Moreover, two or more steps or actions may be conducted simultaneously.

Where a range or list of values is provided, each intervening value between the upper and lower limits of that range or list of values is individually contemplated and is encompassed within the present teachings as if each value were specifically enumerated herein. The listing of exemplary values or ranges is not a disclaimer of other values or ranges between and including the upper and lower limits of a given range.

The scope of the invention is to be defined not by the preceding illustrative description but instead by the following claims, and all changes that come within the meaning and range of equivalency of the claims are intended to be embraced therein.

## Claims

1. A method of predicting response to treatment with a statin, the method comprising:
measuring a proprotein convertase subtilisin kexin type 9 (PCSK9) level in a sample obtained from a patient prior to the patient taking a statin or after a sufficient washout period has elapsed for the patient,
wherein a measured PCSK9 level falling within a predetermined target range is indicative that the patient is likely to respond favorably to treatment with a statin, and
wherein a measured PCSK9 level above the predetermined range is indicative that the patient is likely to have an attenuated response to treatment with a statin.

2. The method of claim 1, wherein the predetermined target range is between about 0 nM to about 7 nM.

3. The method of claim 1, wherein the measured PCSK9 level is an absolute concentration.

4. The method of claim 1, wherein the measured PCSK9 level is a normalized concentration.

5. The method of claim 1, wherein the sufficient washout period is about 6 weeks.

6. A computer system for predicting patient response to treatment with a statin, the computer system comprising:
an electronic memory device; and
an electronic processor in communication with the memory device, wherein the memory device comprises instructions that when executed by the processor cause the processor to:
query a proprotein convertase subtilisin kexin type 9 (PCSK9) level measured in a sample obtained from a patient prior to the patient taking a statin or after a sufficient washout period has elapsed for the patient;
compare the measured PCSK9 level to a threshold range; and
generate a notification indicative of whether the measured PCSK9 level is within the target range,
wherein a measured PCSK9 level falling within a predetermined target range is indicative that the patient is likely to respond favorably to treatment with a statin, and
wherein a measured PCSK9 level above the predetermined range is indicative that the patient is likely to have an attenuated response to treatment with a statin.

7. The method of any one of claims 1-5, wherein the statin is selected from the group consisting of atorvastatin, cerivastatin, fluvastatin, lovastatin, mevastatin, pitavastatin, pravastatin, rosuvastatin, and simvastatin.

## Patentansprüche

1. Ein Verfahren zum Vorhersagen der Reaktion auf die Behandlung mit einem Statin, wobei das Verfahren Folgendes umfasst:
Messen eines Grades an Proproteinkonvertase Subtilisin/Kexin Typ 9 (PCSK9) in einer Probe, erhalten von einem Patienten, bevor der Patient ein Statin eingenommen hat oder nachdem für den Patienten ein ausreichender Auswaschphasenzeitraum vergangen ist,
wobei ein gemessener Grad an PCSK9, der in einem vorbestimmten Zielbereich liegt, darauf hindeutet, dass der Patient wahrscheinlich positiv auf die Behandlung mit einem Statin ansprechen wird, und
wobei ein gemessener Grad an PCSK9, der über dem vorbestimmten Bereich liegt, darauf hindeutet, dass der Patient wahrscheinlich eine abgeschwächte Reaktion auf die Behandlung mit einem Statin haben wird.

2. Verfahren nach Anspruch 1, wobei der vorbestimmte Zielbereich zwischen etwa 0 nM und etwa 7 nM liegt.

3. Verfahren nach Anspruch 1, wobei der gemessene Grad an PCSK9 in einer absoluten Konzentration vorliegt.

4. Verfahren nach Anspruch 1, wobei der gemessene Grad an PCSK9 in einer normalisierten Konzentration vorliegt.

5. Verfahren nach Anspruch 1, wobei der ausreichende Auswaschphasenzeitraum etwa 6 Wochen beträgt.

6. Ein Computersystem zum Vorhersagen der Reaktion eines Patienten auf die Behandlung mit einem Statin, wobei das Computersystem Folgendes umfasst:
eine elektronische Speichervorrichtung; und
einen elektronischen Prozessor, der mit der Speichervorrichtung in Kommunikation steht, wobei die Speichervorrichtung Anweisungen umfasst, die bei Ausführung durch den Prozessor den Prozessor zu Folgendem veranlassen:
Abfragen eines Grades an Proproteinkonvertase Subtilisin/Kexin Typ 9 (PCSK9) in einer Probe, erhalten von einem Patienten, bevor der Patient ein Statin eingenommen hat oder nachdem für den Patienten ein ausreichender Auswaschphasenzeitraum vergangen ist;
Vergleichen des gemessenen Grades an PCSK9 mit einem Schwellenbereich; und Erzeugen einer Meldung, die anzeigt, ob der gemessene Grad an PCSK9 innerhalb des Zielbereichs liegt,
wobei ein gemessener Grad an PCSK9, der in einem vorbestimmten Zielbereich liegt, darauf hindeutet, dass der Patient wahrscheinlich positiv auf die Behandlung mit einem Statin ansprechen wird, und
wobei ein gemessener Grad an PCSK9, der über dem vorbestimmten Bereich liegt, darauf hindeutet, dass der Patient wahrscheinlich eine abgeschwächte Reaktion auf die Behandlung mit einem Statin haben wird.

7. Verfahren nach einem der Ansprüche 1-5, wobei das Statin ausgewählt ist aus der Gruppe, bestehend aus Atorvastatin, Cerivastatin, Fluvastatin, Lovastatin, Mevastatin, Pitavastatin, Pravastatin, Rosuvastatin und Simvastatin.

## Revendications

1. Un procédé de prédiction de réaction à un traitement avec une statine, le procédé comprenant :
mesurer un niveau de proprotéine convertase subtilisine/kexine type 9 (PCSK9) dans un échantillon obtenu chez un patient avant la prise d'une statine par le patient ou après l'écoulement d'une période de rinçage suffisante pour le patient,
dans lequel le fait pour un niveau de PCSK9 mesuré de tomber dans une fourchette cible prédéterminée indique que le patient est susceptible de réagir favorablement au traitement avec une statine, et
dans lequel un niveau de PCSK9 mesuré supérieur à la fourchette prédéterminée indique que le patient est susceptible d'avoir une réaction atténuée au traitement avec une statine.

2. Le procédé de la revendication 1, dans lequel la fourchette cible prédéterminée va d'environ 0 nM à environ 7 nM.

3. Le procédé de la revendication 1, dans lequel le niveau de PCSK9 mesuré est une concentration absolue.

4. Le procédé de la revendication 1, dans lequel le niveau de PCSK9 mesuré est une concentration normalisée.

5. Le procédé de la revendication 1, dans lequel la période de rinçage suffisante est d'environ 6 semaines.

6. Un système informatique pour prédire la réaction d'un patient à un traitement avec une statine, le système informatique comprenant :
un dispositif de mémoire électronique ; et
un processeur électronique en communication avec le dispositif de mémoire, dans lequel le dispositif de mémoire comprend des instructions qui, quand elles sont exécutées par le processeur, amènent le processeur à :
rechercher un niveau de proprotéine convertase subtilisine/kexine type 9 (PCSK9) mesuré dans un échantillon obtenu chez un patient avant la prise d'une statine par le patient ou après l'écoulement d'une période de rinçage suffisante pour le patient ;
comparer le niveau de PCSK9 mesuré à une fourchette seuil ; et
générer une notification qui indique si le niveau de PCSK9 mesuré se situe à l'intérieur de la fourchette cible,
dans lequel le fait pour un niveau de PCSK9 mesuré de tomber dans une fourchette cible prédéterminée indique que le patient est susceptible de réagir favorablement au traitement avec une statine, et
dans lequel un niveau de PCSK9 mesuré supérieur à la fourchette prédéterminée indique que le patient est susceptible d'avoir une réaction atténuée au traitement avec une statine.

7. Le procédé de l'une quelconque des revendications 1 à 5, dans lequel la statine est sélectionnée dans le groupe composé de l'atorvastatine, la fluvastatine, la lovastatine, la mévastatine, la pitavastatine, la pravastatine, la rosuvastatine, et la simvastatine.


### SEQUENCE LISTING

<110> BG Medicine, Inc.
   Muntendam, Pieter
<120> METHODS FOR PREDICTING CARDIOVASCULAR EVENTS AND MONITORING TREATMENT USING PCSK9
<130> BYG-041PC
<150> US 61/314,316
   <151> 2010-03-16
<160> 3
<170> PatentIn version 3.5
<210> 1
   <211> 15
   <212> PRT
   <213> Homo sapiens
<400> 1
   Cys Ala Pro Asp Glu Glu Leu Leu Ser Cys Ser Ser Phe Ser Arg
   1               5                   10                  15
<210> 2
   <211> 13
   <212> PRT
   <213> Homo sapiens
<400> 2
   Asp Val Ile Asn Glu Ala Trp Phe Pro Glu Asp Gln Arg
   1               5                   10
<210> 3
   <211> 15
   <212> PRT
   <213> Homo sapiens
<400> 3
   Ile Leu His Val Phe His Gly Leu Leu Pro Gly Phe Leu Val Lys
   1               5                   10                  15